# EUROPEAN PATENT APPLICATION

(11) **EP 3 824 880 A1**
(43) Date of publication of application: **26.05.2021**
(21) Application number: 19211244.9
(22) Date of filing: 25.11.2019
(51) Int. Cl.: A61K 9/19, A61K 9/06, A61K 31/145, A61K 47/26, A61K 47/34, A61P 17/16, A61K 9/00

(54) **FREEZE-DRIED POWDER CONTAINING 2-[(3-AMINOPROPYL)AMINO]ETHANETHIOL AND ITS USE FOR PREPARING A THERMOGEL**

(71) Applicant: Clevexel Pharma, 75013 Paris (FR); Institut Gustave Roussy, 94800 Villejuif (FR)
(72) Inventor: BRICOUT, Denis, 94430 Chennevieres-sur-Marne (FR); WANG-ZHANG, Xiuping, 75013 PARIS (FR); DEUTSCH, Eric, 75011 PARIS (FR); CLEMENSON, Céline, 91300 MASSY (FR)
(74) Representative: Agasse, Stéphane

(57) **Abstract**

A freeze-dried powder for preparing a thermogel comprising:
- from 1% to 35% of 2-[(3-aminopropyl)amino]ethanethiol or one of its pharmaceutically acceptable salt;
- from 40% to 85% of one or more poloxamer; and
- from 0,1% to 20% of one or more carbohydrate compound.

## Description

The present invention relates to a freeze-dried powder containing 2-[(3-aminopropyl)amino]ethanethiol and its use for preparing a thermogel.

Amifostine (S-{2-[(3-aminopropyl)amino]ethyl}dihydrogenophosphorothioate), also known as ethiofos, is a phosphorylated prodrug that is converted into free active 2-[(3-aminopropyl)amino]ethanethiol (amifostine thiol) under the action of the alkaline phosphatase.

Amifostine is a cytoprotective adjuvant used in cancer chemotherapy and radiotherapy involving DNA-binding chemotherapeutic agents. To the date, it is marketed by Clinigen Group under the trade name Ethyol® as a sterile lyophilized powder for IV perfusion for the following indications:
- reduction of the cumulative renal toxicity associated with repeated administration of cisplatin in patients with advanced ovarian cancer; and
- reduction of the incidence of moderate to severe xerostomia in patients undergoing post-operative radiation treatment for head and neck cancer, where the radiation port includes a substantial portion of the parotid glands.

Other possible uses of amifostine or amisfotine thiol have been reported over the years. For example, Montana et al. tested the effect of amifostine applied topically to protect intestinal mucosa in patients undergoing radiation treatment of the pelvis ("Topical Application of WR-2721 to Prevent Radiation-Induced Proctosigmoiditis", Cancer, 69(11), 2826-2830 (1992)). Similarly, Uzal et al. reported the protective effect of amifostine on radiation-induced proctitis when topically administered in high doses by intrarectal route ("The Protective Effect of Amifostine on Radiation-Induced Proctitis: Systemic Versus Topical Application", Balkan Med J 29, 32-38 (2012)).

Clemenson et al. demonstrated that a thermogel containing thiol metabolite of amifostine (CPh-1014) greatly reduced the severity of oral mucositis and dermatitis induced by irradiation in *in vivo* mouse models in comparison to a thermogel containing amifostine ("Preventing Radiation-Induced Injury by Topical Application of an Amifostine Metabolite-Loaded Thermogel", Int J Radiation Oncol Biol Phys, Vol. 104, No. 5, pp. 1141-1152 (2019)).

US 6,239,119 discloses methods of treating or protecting mucosal tissue from damages associated with radiation and/or chemotherapeutic treatment of cancers, by the topical application of amifostine and related compounds. The use of oral gel formulations for treating mucosal tissues within the oral cavity is reported.

The main issue encountered with formulations containing amifostine or a derivative thereof, including gel or thermogel, resides in its lack of stability. This lack of stability leads to the degradation of the active into various impurities, mainly into symmetrical disulfide impurity.

International patent application WO-A-2018/100008 discloses a process for the preparation of freeze-dried 2-[(3-aminopropyl)amino]ethanethiol formulation allowing preparing a thermogel, said freeze-dried formulation being easy to reconstitute and stable during its storage. Despite the process disclosed in that patent application significantly increases the stability of amifostine thiol during the storage by limiting apparition of the disulfide impurity in comparison to previously known formulations, such compositions need to be stored at low temperatures (5°C or less) to ensure that amifostine thiol does not significantly degrade into disulfide and other impurities.

To date, there remains a need for a freeze-dried powder containing 2-[(3-aminopropyl)amino]ethanethiol that can be easily reconstituted for preparing a thermogel and that is stable when stored at ambient conditions (i.e. temperature and humidity).

It has now surprisingly been found that a freeze-dried powder containing 2-[(3-aminopropyl)amino]ethanethiol, a poloxamer and a carbohydrate compound can be easily reconstituted for preparing a thermogel and is stable during months even though it is stored at ambient conditions.

Accordingly, the present invention relates to a freeze-dried powder for preparing a thermogel comprising:
- from 1% to 35% of 2-[(3-aminopropyl)amino]ethanethiol or one of its pharmaceutically acceptable salt;
- from 40% to 85% of one or more poloxamer; and
- from 0,1% to 20% of one or more carbohydrate compound.

Freeze-dried powder according to the present invention can be stored for months at ambient temperature without observing significant degradation of 2-[(3-aminopropyl)amino]ethanethiol into disulfide impurity. In addition, it can be easily reconstituted for obtaining a thermogel that can be used for treating or protecting mucosal or cutaneous tissue from damage associated with anticancer therapy, in particular with radiation and/or chemotherapeutic treatment of cancers, by topical application.

In the context of the present invention:
- "freeze-dried powder" (or "lyophilised powder") designates any powder containing less than 8% of water, preferably less than 5% of water, still preferably less than 2% of water;
- "2-[(3-aminopropyl)amino]ethanethiol" designates the active cytoprotective thiol metabolite of amifostine, also designated by "amifostine thiol" or "WR-1065";
- "poloxamer" designates non-ionic block copolymers of ethylene oxide (EO) and propylene oxide (PO) synthesized by sequential addition of propylene oxide first and then ethylene oxide to a low molecular weight water-soluble propylene glycol (ref. G. Bonacucina et al., "Thermosensitive Self-Assembling Block Copolymers as Drug Delivery Systems", Polymers 2011, 3, 779-811). Poloxamer are commercialised under various names among which Pluronics®, Lutrol®, Kolliphor®;
- "carbohydrate compound" designates any biomolecule having the following formula: Cₘ(H₂O)ₙ (where m may be different from n). Carbohydrate compounds are also known as "saccharides". Preferably, "carbohydrate compound" designates monosaccharide or disaccharide, i.e. sugars;
- "thermogel" (or "thermosensitive gel") designates any polymer solution exhibiting the property of transitioning between a liquid and a firm gel during temperature transition. Preferably, "thermogel" designates an hydrogel which is liquid at room temperature, and gels at body temperature and capable of adhering to the mucous membrane and skin by its gelled state;
- "pharmaceutically acceptable salt" of an active ingredient designates any salt of addition of said active ingredient with an inorganic or organic acid by the action of such an acid within an organic or aqueous solvent, such as an alcohol, a ketone, an ether or a chlorinated solvent, and which is acceptable from a pharmaceutical point of view; and
- unless otherwise specified, all % values are weight %.

The present invention relates a freeze-dried powder for preparing a thermogel comprising 2-[(3-aminopropyl)amino]ethanethiol, a poloxamer and a carbohydrate compound. Preferably, the present invention relates to a freeze-dried powder for preparing a thermogel as defined above possessing the following characteristics, taken individually or in combination:
- the freeze-dried powder contains from 5% to 30% of 2-[(3-aminopropyl)amino]ethanethiol; more preferably from 10% to 20% of 2-[(3-aminopropyl)amino]ethanethiol;
- the freeze-dried powder contains from 45% to 80% of poloxamer; more preferably from 60% to 75% of poloxamer;
- the freeze-dried powder contains one or more poloxamer chosen as being Poloxamer 407 or Poloxamer 188. More preferably, the the freeze-dried powder contains a mixture of Poloxamer 407 and Poloxamer 188. Even more preferably, the freeze-dried powder contains a mixture of Poloxamer 407 and Poloxamer 188 in a Poloxamer 407 : Poloxamer 188 weight ratio of from 1.5 to 6;
- the freeze-dried powder contains from 0,5% to 15% of a carbohydrate compound; more preferably from 1% to 10% of carbohydrate compound; and/or
- the freeze-dried powder contains one or more carbohydrate compound chosen among sugars. More preferably, the freeze-dried powder contains one or more carbohydrate compound chosen among mannitol, lactose, sucrose, trehalose, sorbitol, glucose or raffinose. Even more preferably, the freeze-dried powder contains one or more carbohydrate compound chosen as being sorbitol or mannitol.

The freeze-dried powder according to the present invention may also contain further ingredients, such as flavouring agents or high-density sweeteners. Accordingly, the present invention also relates to a freeze-dried powder for preparing a thermogel as defined above further containing one or more of the following ingredients:
- a flavouring agent such as apple, banana, cherry, coconut lemon, menthol, orange, raspberry, strawberry, tutti frutti or vanilla; and/or
- a high-intensity sweetener such as saccharin, aspartame, acesulfame potassium, sucralose or neotame.

The freeze-dried powder according to the present invention may be prepared according to any process known by skilled artisan. In particular, the present invention also relates to a process for preparing a freeze-dried powder as defined above, said process comprising the following steps:
- hydrolysing amifostine into amifostine thiol under acidic conditions;
- preparing a solution containing the excipients by:
   ∘ dissolving and mixing firstly all excipients presented in the formulation, except the poloxamer(s);
   ∘ cooling the temperature before adding gradually the polaxamer(s) to get an homogenous excipient solution;
- mixing the obtained amifostine thiol solution with the obtained excipient solution ;
- freeze-drying the obtained solution; and
- nitrogen inerting before sealing.

As explained above, the freeze-dried powder according to the present invention can be used for preparing a thermogel useful for treating or protecting mucosal or cutaneous tissue from damage associated with anticancer therapy, in particular with radiation and/or chemotherapeutic treatment of cancers, by topical application. Accordingly, the present invention also relates to a process for preparing a thermogel comprising the reconstitution of the freeze-dried powder as defined above with an aqueous solution.

The aqueous solution used for preparing the thermogel may be made of water only or contain one or further ingredient such as a poloxamer, penetration enhancer, taste masking agents such as aroma or sweetener, mucoadhesive agent, cosolvents, humectant or colouring agent.

The aqueous solution may be added to the freeze-dried powder according to the present invention in a freeze-dried powder: aqueous solution (w/w) ratio of from 1 to 5.

The thermogel prepared according to the above process is novel. Accordingly, the present invention also relates to a thermogel obtainable by a process as described above.

The thermogel according to the present invention may be used for treating or protecting mucosal or cutaneous tissue from damage associated with anticancer therapy, in particular with radiation and/or chemotherapeutic treatment of cancers, by topical application. Accordingly, the present invention also relates to a thermogel as defined above for treating or protecting mucosal or cutaneous tissue from damage associated with anticancer therapy, in particular with radiation and/or chemotherapeutic treatment of cancers.

As damages associated with radiation treatment of cancers, one may cite oral mucositis (in case of radiation of head and neck cancer), epithelitis in the neck (in case of radiation of head and neck cancer), esophagitis (in case of radiation of the lung), cutaneous erythema (in case of radiation of breast), enteritis (in case of abdominal irradiation), vaginitis and vaginal dryness (in case of pelvic irradiation), rectitis (in case of pelvic irradiation), and loss of hair (in case of brain irradiation). Preferably, the present invention relates to a thermogel as defined above for treating radiation-induced oral mucositis or cutaneous erythema.

Finally, the present invention also relates to a method of treating or protecting mucosal or cutaneous tissue from damage associated with anticancer therapy, in particular with radiation and/or chemotherapeutic treatment of cancers by topical application of the thermogel as defined above. Preferably, the present invention relates to a method of treating radiation-induced oral mucositis or cutaneous erythema by topical application of the thermogel as defined above.

The present invention will now be illustrated in a non-limiting manner by the following examples.

### Example 1 - Freeze-dried composition

### 1.1 - Process for preparing freeze-dried composition

A solution of amifostine at 500 mg/ml is prepared in hydrochloric acid 4M and heated in a water-bath at 60°C for 1 hour to obtain the amifostine thiol solution.

A solution containing the excipients is prepared by:
- dissolving and mixing firstly all excipients presented in the formulation, except the poloxamers, in a bottle containing water under magnetic agitation;
- cooling the temperature of the obtained solution by placing the bottle in ice and keeping it cold for next step; and
- adding poloxamers (Kolliphor) gradually, under magnetic agitation, until complete solubilisation.

The obtained solution of amifostine thiol and the excipient solution are mixed at required proportion to get the solution at target composition and concentration.

Solution is then distributed in 20 ml vials and is freeze-dried using the program reported in Table1 below.

**Table 1: Freeze-drying Program**

| **Step** | **Product Temp. (°C)** | **Pressure (µbar)** | **Ramp (°C/min)** | **Hold (H)** |
|---|---|---|---|---|
| Loading | 5 | / | / | / |
| Freezing Segment 1 | -5 | / | 0.5 | 1 |
| Freezing Segment 2 | -40 | / | 1 | 5 |
| Primary drying | -18 | 200 | 0.03 | 48 |
| Secondary drying | 20 | 50 | NA | 4 |

The obtained freeze-dried powder is then inerted under nitrogen manually during 30 seconds before sealing using rubber stopper and aluminium crimp.

### 1.2 - Freeze-dried powders 1 and 2

Composition of freeze-dried powders 1 and 2 (LP1 and LP2) reported in Table 2 below have been prepared according to the process disclosed above (example 1.1).

**Table 2 - LP1 and LP2**

| **Ingredient** | **Freeze-dried powder 1-LP1 (% w/w)** | **Freeze-dried powder 2 - LP2 (% w/w)** |
|---|---|---|
| Amifostine thiol | 15.8% | 16.2% |
| Kolliphor P407 | 45.5% | 46.7% |
| Kolliphor P188 | 17.8% | 18.2% |
| Sorbitol | 5.1% | - |
| Mannitol | - | 2.6% |
| Hydrochloric acid | 4.4% | 4.5% |
| Phosphoric acid | 11.5% | 11.8% |

The obtained white freeze-dried powders LP1 and LP2 have good appearance and well-formed cake.

### Example 2 - Preparation of thermogel

To reconstitute freeze-dried powders LP1 and LP2 to thermogel, 5 ml of water are introduced to the sealed vial through the stopper using a needle and a syringe.

After waiting one minute to let water penetrating in the cake, the vial is then gently shake by hand for two minutes to get a clear thermogel.

### Example 3 - Stability of freeze-dried powders

### 3.1 - Reference

A freeze-dried powder which does not contain carbohydrate compound and which has been prepared according to process disclosed in example 1.1 is used as a reference (i.e. Ref.) for this evaluation. Composition of said reference freeze-dried powder is reported in the Table 3 below.

**Table 3 - Reference**

| **Ingredient** | **Reference freeze-dried powder - Ref. (% w/w)** |
|---|---|
| Amifostine thiol | 16.6% |
| Kolliphor P407 | 47.9% |
| Kolliphor P188 | 18.7% |
| Hydrochloric acid | 4.6% |
| Phosphoric acid | 12.1% |

### 3.2 - Experimental protocol

Vials of LP1, LP2 and Ref. were stored under the following conditions:
- 5°C / Ambient RH (i.e. relative humidity);
- 25°C / 60%RH; and
- 40°C / 75%RH;
in stability chambers.

Stability of LP1, LP2 and Ref. under these conditions was assessed after 1-, 3- and 6-month storage.

Stability testing was performed according to ICH guideline *"Q1A(R2) Stability Testing of New Drug Substances and Products".* The following parameters have been assessed:
- cake appearance by visual inspection;
- solution appearance by visual inspection;
- pH using pH-meter;
- purity; and impurity by HPLC.

### 3.3 - Results

Results obtained for LP1, LP2 and Ref. are reported in Tables 4 to 6 below.

**Table 4 - LP1 stability**

| **Storage conditions** | **T₀** | **1-month** | | | **3-months** | | | **6-months** | | |
|---|---|---|---|---|---|---|---|---|---|---|
| | | **5°C** | **25°C/65 %RH** | **40°C/75 %RH** | **5°C** | **25°C/65 %RH** | **40°C/75 %RH** | **5°C** | **25°C/65 %RH** | **40°C/75 %RH** |
| Cake appearance | White cake | White cake | White cake | White cake | White cake | White cake | White cake | White cake | White cake | White cake |
| Solution appearance | Clear | Clear | Clear | Clear | Clear | Clear | Clear | Clear | Clear | Clear |
| pH | 4.5 | 4.5 | 4.5 | 4.5 | 4.4 | 4.5 | 4.4 | 4.4 | 4.5 | 4.5 |
| Purity (area% at 210 nm) | **99.3** | **99.5** | **99.1** | **99.2** | **99.6** | **99.2** | **99.2** | **99.6** | **99.5** | **99.2** |
| *Impurity (area% at 210 nm)* | | | | | | | | | | |
| *Amifostine* | *0.05* | *0.06* | *0.10* | *0.070* | *0.06* | *0.10* | *0.05* | *0.08* | *0.08* | *<0.05* |
| *Disulfide* | *0.36* | *0.13* | *0.25* | *.28* | *0.18* | *0.29* | *0.35* | *0.16* | *0.16* | *0.21* |

**Table 5 - LP2 stability**

| **Storage conditions** | **T₀** | **1-month** | | | **3-months** | | | **6-months** | | |
|---|---|---|---|---|---|---|---|---|---|---|
| | | **5°C** | **25°C/65 %RH** | **40°C/75 %RH** | **5°C** | **25°C/65 %RH** | **40°C/75 %RH** | **5°C** | **25°C/65 %RH** | **40°C /75 %RH** |
| Cake appearance | White: cake | White cake | White cake | White cake | White cake | White cake | White cake | White cake | White cake | White cake |
| Solution appearance | Clear | Clear | Clear | Clear | Clear | Clear | Clear | Clear | Clear | Clear |
| pH | 4.6 | 4.5 | 4.5 | 45 | 4.4 | 4.4 | 4.4 | 4.4 | 4.4 | 4.5 |
| Purity (area% at 210 nm) | **99.6** | **99.5** | **99.4** | **99.1** | **99.4** | **99.3** | **98.9** | **99.6** | **99.4** | **98.8** |
| *Impurity (area% at 210 nm)* | | | | | | | | | | |
| *Amifostine* | *<0.05* | *0.06* | *0.10* | *0.12* | *0.07* | *0.09* | *0.08* | *0.08* | *0.11* | *0.07* |
| *Disulfide* | *0.13* | *0.13* | *0.15* | *0.18* | *0.23* | *0.23* | *0.41* | *0.14* | *0.11* | *0.18* |

**Table 6 - Ref. stability**

| **Storage conditions** | **T₀** | **1-month** | | | **3-months** | | | **6-months** | | |
|---|---|---|---|---|---|---|---|---|---|---|
| | | **5°C** | **25°C/65 %RH** | **40°C/75 %RH** | **5°C** | **25°C/65 %RH** | **40°C/75 %RH** | **5°C** | **25°C/65 %RH** | **40°C/75 %RH** |
| Cake appearance | White cake | White cake | White cake | White cake | White cake | White cake | White cake | White cake | White cake | White cake |
| Solution appearance | Clear | Clear | Clear | Clear | Clear | Clear | Clear | Clear | Clear | Clear |
| pH | 4.6 | 4.7 | 4.6 | 4.6 | 4.7 | 4.6 | 4.6 | 4.6 | 4.6 | 4.7 |
| Purity (area% at 210 nm) | | 99.5 | 99.1 | 99.0 | 99.6 | 99.2 | 98.6 | 98.9 | 99.2 | 95.4 |
| *Impurity (area% at 210 nm)* | | | | | | | | | | |
| *Amifostine* | *<0.05* | *0.05* | *0.13* | *0.12* | *0.07* | *0.10* | *0.11* | *0.09* | *0.13* | *<0.05* |
| *Disulfide* | *0.16* | *0.15* | *0.25* | *0.24* | *0.17* | *0.39* | *0.50* | *0.23* | *0.21* | *2.65* |

### 3.4 - Conclusion

Reference formulation Ref. (which does not contain carbohydrate compound) is instable when it is stored at accelerated condition 40°C/75%RH. The key degradation impurity disulfide (RRT 1.25) was particularly increased (> 2.6%) after 6-months storage at 40°C/75%RH.

On the contrary, stability of LP1 and LP2 is excellent, even after 6 months storage at accelerated condition (40°C/75%RH). Purity determination by HPLC has showed low level of the key impurities, amifostine and its disulfide.

Furthermore, it can be noted that addition of carbohydrate compound, in particular mannitol (see LP2) in freeze-dried composition is beneficial to reconstitution. The reconstitution of the product containing mannitol is fast and no significant change is observed after 6-months storage even at 40°C/75%RH.

## Claims

**1.** A freeze-dried powder for preparing a thermogel comprising:
- from 1% to 35% of 2-[(3-aminopropyl)amino]ethanethiol or one of its pharmaceutically acceptable salt;
- from 40% to 85% of one or more poloxamer; and
- from 0,1% to 20% of one or more carbohydrate compound.

**2.** A freeze-dried powder according to claim 1, **characterised in that** it contains from 5% to 30% of 2-[(3-aminopropyl)amino]ethanethiol.

**3.** A freeze-dried powder according to claim 1 or 2, **characterised in that** it contains from 45% to 80% of poloxamer.

**4.** A freeze-dried powder according to claim 3, **characterised in that** it contains from 60% to 75% of poloxamer.

**5.** A freeze-dried powder according to any of claims 1 to 4, **characterised in that** poloxamer is chosen as being Poloxamer 407 or Poloxamer 188.

**6.** A freeze-dried powder according to any of claims 1 to 5, **characterised in that** it contains a mixture of Poloxamer 407 and Poloxamer 188.

**7.** A freeze-dried powder according to any of claims 1 to 6, **characterised in that** it contains from 0,5% to 15% of a carbohydrate compound.

**8.** A freeze-dried powder according to claim 7, **characterised in that** it contains from 1% to 10% of carbohydrate compound.

**8.** A freeze-dried powder according to any of claims 1 to 7, **characterised in that** carbohydrate compound is chosen among sugars.

**9.** Freeze-dried powder according to claim 8, **characterised in that** carbohydrate compound is chosen among mannitol, lactose, sucrose, trehalose, sorbitol, glucose or raffinose.

**10.** Freeze-dried powder according to claim 9, **characterised in that** carbohydrate compound is chosen as being sorbitol or mannitol.

**11.** A freeze-dried powder according to any of claims 1 to 10, **characterised in that** it further contains one or more of the following ingredients:
- flavouring agent such as apple, banana, cherry, coconut, lemon, menthol, orange, raspberry, strawberry, coconut, tutti frutti or vanilla; and/or
- high-intensity sweetener such as saccharin, aspartame, acesulfame potassium, sucralose or neotame.

**12.** A process for preparing a freeze-dried powder according to any of claims 1 to 11, said process comprising the following steps:
comprising the following steps:
- hydrolysing amifostine into amifostine thiol under acidic conditions;
- preparing a solution containing the excipients by:
∘ dissolving and mixing firstly all excipients presented in the formulation, except the poloxamer(s);
∘ cooling the temperature before adding gradually the polaxamer(s) to get a homogenous excipient solution;
- mixing the obtained amifostine thiol solution with the obtained excipient solution ;
- freeze-drying the obtained solution; and
- nitrogen inerting before sealing.

**13.** A process for preparing a thermogel comprising the reconstitution of the freeze-dried powder according to any of claims 1 to 11 with an aqueous solution.

**14.** A thermogel obtainable by a process according to claim 13.

**15.** A thermogel according to claim 14 for treating or protecting mucosal or cutaneous tissue from damage associated with anticancer therapy, in particular with radiation and/or chemotherapeutic treatment of cancers.
